# EUROPEAN PATENT APPLICATION

(11) **EP 4 545 650 A1**
(43) Date of publication of application: **30.04.2025**
(21) Application number: 24208844.1
(22) Date of filing: 25.10.2024
(51) Int. Cl.: C12Q 1/686, C12Q 1/689

(54) **ADDITIVE FOR PCR AMPLIFICATION, MULTIPLEX PCR AMPLIFICATION KIT, AND USE THEREOF**

(30) Priority: 27.10.2023 CN 202311422098
(71) Applicant: GeneMind Biosciences Company Limited, Shenzhen City, Guangdong 518023 (CN)
(72) Inventor: JU, Mingxia, Shenzhen City, 518023 (CN); GAO, Yifan, Shenzhen City, 518023 (CN); ZENG, Lidong, Shenzhen City, 518023 (CN); ZHOU, Letian, Shenzhen City, 518023 (CN); KANG, Huanhuan, Shenzhen City, 518023 (CN)
(74) Representative: CH Kilger Anwaltspartnerschaft mbB

(57) **Abstract**

The present disclosure provides an additive for PCR amplification, a multiplex PCR amplification kit, and use thereof, wherein the additive comprises benzalkonium bromide. The additive, when added to a simplex PCR system or a multiplex PCR system, can provide great promoting effects on the amplification efficiency and specificity of the simplex PCR amplification or multiplex PCR amplification, and thus possess great application prospects in simplex PCR or multiplex PCR. The additive may be used in combination with tetramethylammonium chloride (TMAC) and/or tetrapolyethylene glycol monolauryl ether (Brij 4L).

## Description

### TECHNICAL FIELD

The present disclosure relates to the technical field of biology, and particularly, to an additive for PCR amplification, a multiplex PCR amplification kit, and use thereof.

### BACKGROUND

At present, tuberculosis has become a serious public health concern throughout the world, featuring a high morbidity and a low detection rate in bacteriological tests; for multidrug-resistant tuberculosis, the conditions are similar. In addition to medical history and physical examination, major diagnostic methods in clinical practice include bacteriological culture (gold standard), molecular biological detection, radiological detection, immunological detection (for latent tuberculosis infection), and pathological detection.

The molecular biological detection can generally be divided into two categories: One is molecular detections specific for tubercle bacillus, including real-time fluorescent quantitative polymerase chain reaction (PCR), semi-nested automatic real-time fluorescent quantitative PCR, loop-mediated isothermal amplification (LAMP), real-time fluorescent nucleic acid isothermal amplification, cross-primer amplification (CPA), and the like. The other one includes amplifying the nucleic acid fragment with a universal PCR primer, sequencing the amplified sample, and analyzing the sequencing data. In addition, many new techniques (metagenomic next-generation sequencing (mNGS), transcriptome, and metabolome) are continuously progressing, and more alternative solutions are provided for molecular biological detection.

Currently, a variety of methods for detecting the drug resistance of tubercle bacillus are present, but in the prior art, the detection effect is mainly improved by optimizing the primer design for tuberculosis multidrug resistance-related genes and optimizing DNA polymerases. In multiplex PCR amplification, with the increase of amplification targets, it is difficult to further improve the detection effects only by optimizing the primer design and DNA polymerase selection. Therefore, it is of great value to develop a PCR amplification additive capable of improving the amplification efficiency and specificity.

### SUMMARY

For the defects in the prior art, the present disclosure is intended to provide an additive for PCR amplification, a multiplex PCR amplification kit, and use thereof. The additive, when added to a multiplex PCR system, can provide promoting effects on the amplification efficiency and specificity of multiplex PCR, and thus possess great application prospects in multiplex PCR. The present disclosure further provides a multiplex PCR amplification kit. The components in the kit can cooperate with each other to achieve excellent amplification effects on target genes. The present disclosure further provides a method for detecting drug resistance in *Mycobacterium tuberculosis* by using the kit. The method features good sequencing uniformity. The M. *tuberculosis* DNA can be successfully amplified and sequenced at a ppm. proportion in the presence of the human genome.

In order to achieve the purpose, the present disclosure adopts the following technical scheme:
In a first aspect, the present disclosure provides an additive for PCR amplification, including benzalkonium bromide (BKB).

Optionally, benzalkonium bromide is present at a concentration of 0.001-0.1% by weight in a PCR amplification system.

Optionally, benzalkonium bromide is present at a concentration of 0.005-0.1% by weight in a PCR amplification system.

Optionally, the additive further includes tetramethylammonium chloride (TMAC) and/or tetrapolyethylene glycol monolauryl ether (Theist).

Optionally, the additive further includes at least one of bovine serum albumin, polyethylene glycol, or dimethyl sulfoxide.

Optionally, the additive is a combination of benzalkonium bromide and tetramethylammonium chloride.

Optionally, the additive is present at concentrations of 0.001-0.1% for benzalkonium bromide and 0.1-0.5% for tetramethylammonium chloride by weight in the PCR amplification system.

Optionally, the additive is a combination of benzalkonium bromide and tetrapolyethylene glycol monolauryl ether.

Optionally, the additive is present at concentrations of 0.001-0.1% for benzalkonium bromide and 0.001-0.2% for tetrapolyethylene glycol monolauryl ether by weight in the PCR amplification system.

Optionally, the additive is a combination of benzalkonium bromide, tetramethylammonium chloride, and tetrapolyethylene glycol monolauryl ether.

Optionally, the additive is present at concentrations of 0.001-0.1% for benzalkonium bromide, 0.1-0.5% for tetramethylammonium chloride, and 0.001-0.2% for tetrapolyethylene glycol monolauryl ether by weight in the PCR amplification system.

Optionally, the additive is for use in multiplex PCR amplification.

In a second aspect, the present disclosure provides a multiplex PCR amplification kit, including the additive for PCR amplification according to the first aspect.

Optionally, the multiplex PCR amplification kit further includes a primer composition.

Optionally, the primer composition includes one or more primer pairs, and the primers are selected from the nucleotide sequences set forth in SEQ ID NOs. 1-174.

Optionally, the primer composition is a nucleotide sequence set forth in SEQ ID NOs. 1-174.

Optionally, the kit further includes a PCR amplification reagent, and the PCR amplification reagent includes: KCI, MgCl₂, Tris-HCl, dNTP, and a DNA polymerase.

In a third aspect, the present disclosure provides use of the multiplex PCR amplification kit according to the second aspect in drug resistance detection in M. *tuberculosis.*

In a fourth aspect, the present disclosure provides a method for detecting drug resistance in M. *tuberculosis,* including:
performing multiplex PCR amplification on a M. *tuberculosis* culture using the multiplex PCR amplification kit according to the second aspect; sequencing the amplification product; and determining the drug resistance of M. *tuberculosis* based on the sequencing result.

The numerical ranges provided herein include not only the points recited above, but also any points between the numerical ranges that are not recited above. For purposes of brevity and clarity, the particular points in the ranges are not exhaustively enumerated.

Compared with the prior art, the present disclosure has the following beneficial effects:
(1) During simplex PCR amplification, the amplification reagent containing benzalkonium bromide, particularly at a concentration of 0.001-0.1% in the amplification system, can improve the amplification efficiency and specificity of the PCR.
(2) During multiplex PCR amplification, the triple additive combination (0.001-0.1% of benzalkonium bromide, 0.1-0.5% of tetramethylammonium chloride, and 0.001-0.2% of tetrapolyethylene glycol monolauryl ether) can improve the amplification efficiency and specificity of the PCR reaction, and when the number of primer pairs in the reaction system is increased to 74 pairs, the primers still work.
(3) The present application further designs a primer combination for determining a tuberculosis drug resistance-related locus, and when the primer combination is combined with the additive combination disclosed herein, a better detection effect is achieved.

### BRIEF DESCRIPTION OF THE DRAWINGS

FIG. 1 illustrates an electrophoretogram of amplified libraries obtained by 13-plex or 74-plex PCR amplification according to examples of the present application.
FIG. 2 illustrates the 74-plex amplification and the library construction results according to examples of the present application.

### DETAILED DESCRIPTION

The embodiments of the present disclosure are further illustrated below by means of specific examples. It will be appreciated by those skilled in the art that the examples are only for the purpose of understanding the present disclosure and should not be construed as limitations to the present disclosure.

The examples with no specified techniques or conditions are performed in accordance with techniques or conditions described in the literature in the art or in accordance with the product instructions. The reagents or instruments provided with no manufacturer specified are conventional and commercially available products.

In the present disclosure, the "additive for PCR amplification" is also referred to as a PCR additive. It is known as a synergist for PCR amplification to improve the specificity of amplification reactions, increase the PCR amplification contents, prevent ineffective amplification, etc., during the PCR amplification. In a PCR amplification process, reagents other than the amplification primers, polymerases, dNTPs or dNTPs, nucleic acid molecule templates, and buffers can be construed as PCR additives.

In the present disclosure, the term "PCR amplification system" refers to a reaction system for PCR amplification, which at least includes all the material elements required for PCR amplification reactions. Generally, the PCR amplification system mainly includes basic elements such as primers, enzymes, dNTPs, templates, Mg²⁺, etc. In the present disclosure, the PCR amplification system includes PCR additives for increasing the amplification efficiency in addition to the above-mentioned common components. The term "present at a concentration by weight in the PCR amplification system" refers to the final concentration of the additive in the reaction solution in which the PCR reaction is carried out.

In the present disclosure, the "nucleic acid molecule" refers to polymeric forms of nucleotides of any length and may include ribonucleotides or analogs thereof, deoxyribonucleotides or analogs thereof, and mixtures of the foregoing nucleotides or analogs thereof. The nucleic acid molecule may refer to a single-stranded polynucleotide or a double-stranded polynucleotide. Nucleotides in a nucleic acid molecule may include natural nucleotides and functionally alternative analogs thereof. Examples of analogs include those that can hybridize to nucleic acids in a sequence-specific manner, or can be used as templates for the replication of particular nucleotide sequences. Natural nucleotides generally have a backbone containing phosphodiester bonds. Analog structures may have alternative backbone linkages including any types known in the art. Natural nucleotides generally have deoxyribose (e.g., found in DNA) or ribose (e.g., found in RNA). Analog structures may have alternative sugar moieties including any types known in the art. Nucleotides may contain natural bases or non-natural bases. Bases in natural DNA may include one or more of adenine, thymine, cytosine, and/or guanine, and bases in natural RNA may include one or more of adenine, uracil, cytosine, and/or guanine. Any non-natural base or base analog may also be used in nucleotides, such as a locked nucleic acid (LNA) and a bridged nucleic acid (BNA).

In the present disclosure, the term "primer" refers to an oligonucleotide or nucleic acid molecule that can hybridize to a target sequence of interest. In one embodiment, the primer serves as a substrate onto which nucleotides may be polymerized by a polymerase. For example, a primer may be used as a starting point for DNA or RNA synthesis to synthesize a new nucleic acid strand starting from its 3' terminus. For example, a sequencing primer can hybridize to a synthesized nucleic acid template strand so as to trigger the synthesis of a new strand complementary to the synthesized nucleic acid template strand. The primer may include any combination of nucleotides or analogs thereof. In some examples, the primer is a single-stranded oligonucleotide or polynucleotide. A set of primers typically includes two artificially synthesized oligonucleotide sequences, with one primer being complementary to one DNA template strand at one terminus of the target region and the other primer being complementary to the other DNA template strand at the other terminus of the target region.

In the present disclosure, the term "sequencing" may also be referred to as "nucleic acid sequencing" or "gene sequencing". The three are used interchangeably and refer to the determination of the type and order of bases or nucleotides (including nucleotide analogs) in a nucleic acid molecule. The sequencing involves the process of binding nucleotides to a template and acquiring the corresponding signals emitted by the nucleotides (including analogs). Sequencing can be performed through a sequencing platform, which may be selected from, but is not limited to, the Hiseq/Miseq/Nextseq/Novaseq sequencing platform (Illumina), the Ion Torrent platform (Thermo Fisher/Life Technologies), the BGISEQ and MGISEQ platforms (BGI) and single-molecule sequencing platforms. The sequencing method may be selected from single-read sequencing and paired-end sequencing. The obtained sequencing results/data (i.e., read fragments) are referred to as reads.

In the present disclosure, "the primer composition includes one or more primer pairs, and the primers are selected from the nucleotide sequences set forth in SEQ ID NOs. 1-174", where one primer pair includes an upstream primer and a downstream primer for the same target nucleic acid molecule, and the primers in the primer composition are present in primer pairs. Illustratively, the "two primer pairs" refers to two sets of upstream and downstream primers for two target nucleic acid molecules or two different target regions of one target nucleic acid molecule in PCR amplification, and the "three primer pairs" refers to three sets of upstream and downstream primers for three target nucleic acid molecules or three different target regions of one or two target nucleic acid molecules in PCR amplification, and so on for understanding primer pairs of other numbers.

In a first aspect, the embodiments of the present disclosure provide an additive for PCR amplification, including benzalkonium bromide.

In the embodiments of the present disclosure, benzalkonium bromide serves as the additive for PCR amplification. On one hand, benzalkonium bromide can reduce the formation of secondary structures of DNA molecules and improve the amplification efficiency of PCR reactions; on the other hand, benzalkonium bromide, as a surfactant, can reduce sodium dodecyl sulfate (SDS) residues used in the PCR amplification reaction process, and reduce the inhibitory effect of SDS on the activity of DNA polymerases in PCR reactions, thereby improving the activity of DNA polymerases and finally improving the PCR amplification efficiency. In addition, researchers found that the addition of benzalkonium bromide to a multiplex PCR amplification reaction system can also improve the amplification specificity of multiplex PCR to some extent.

In the embodiments of the present disclosure, the content of benzalkonium bromide in the PCR amplification reaction system has a certain influence on the improvement of the PCR amplification reaction efficiency by benzalkonium bromide. In some embodiments, benzalkonium bromide is present at a concentration of 0.001-0.1% by weight in a PCR amplification system. It will be appreciated that, in the embodiments of the present application, the concentration by weight refers to the proportion of the mass of the target substance in the total mass of the PCR amplification reaction system. When benzalkonium bromide is present at a concentration of 0.001-0.1% by weight in a PCR amplification system., benzalkonium bromide can effectively improve the PCR amplification reaction efficiency. If the concentration thereof by weight is less than 0.001%, the effect may not be sufficient; as the concentration of benzalkonium bromide by weight increases, the PCR amplification reaction efficiency also increases. However, considering that benzalkonium bromide at a high content may lead to non-specific amplification in multiplex PCR and affect the amplification specificity of multiplex PCR, the concentration of benzalkonium bromide by weight in the PCR amplification system is 0.001-0.1% in the embodiments of the present application. Illustratively, the concentration of benzalkonium bromide by weight in the PCR amplification system may be 0.001%, 0.002%, 0.003%, 0.004%, 0.005%, 0.006%, 0.007%, 0.008%, 0.009%, 0.01%, 0.015%, 0.02%, 0.025%, 0.03%, 0.035%, 0.04%, 0.045%, 0.05%, 0.055%, 0.06%, 0.065%, 0.07%, 0.075%, 0.08%, 0.085%, 0.09%, 0.095%, 0.1%, or the like. In some embodiments, benzalkonium bromide is present at a concentration of 0.005-0.1% by weight in a PCR amplification system.

In some embodiments, the additive further includes tetramethylammonium chloride and/or tetrapolyethylene glycol monolauryl ether. Tetramethylammonium chloride can increase the specificity of the binding of a primer to a nucleic acid template, improve the melting temperature of DNA molecules obtained via the binding, and reduce the base mismatches in a PCR amplification process. Thus, tetramethylammonium chloride can reduce non-specific amplification in a PCR amplification process. Tetrapolyethylene glycol monolauryl ether, as a surfactant, can improve the activity of DNA polymerases and the amplification efficiency of PCR reactions by reducing the formation of the secondary structure of DNA molecules and/or reducing SDS residues in the PCR amplification process. The effect of tetrapolyethylene glycol monolauryl ether is influenced to a certain extent by the addition amount. The effect of improving the PCR amplification reaction efficiency is proportional to the content of tetrapolyethylene glycol monolauryl ether, but in the multiplex PCR amplification, non-specific amplification may be introduced as the concentration by weight increases.

In the embodiments of the present disclosure, the addition of at least one of tetramethylammonium chloride and tetrapolyethylene glycol monolauryl ether along with benzalkonium bromide as the additive for PCR amplification, particularly multiplex PCR amplification, may produce a synergistic effect in a certain way, and exhibit effects of improving the PCR amplification reaction efficiency and/or the amplification specificity of multiplex PCR to a certain extent.

In one implementation, the additive is a combination of benzalkonium bromide and tetramethylammonium chloride. In such a condition, benzalkonium bromide can improve the amplification efficiency, and tetramethylammonium chloride can improve the amplification specificity and reduce the non-specific amplification possibly caused by the increase of benzalkonium bromide content to a certain extent. Benzalkonium bromide and tetramethylammonium chloride can balance the amplification efficiency of PCR reactions and the PCR amplification specificity via their complementation, and jointly improve the amplification efficiency of PCR reactions and the PCR amplification specificity.

In one embodiment, the additive is present at concentrations of 0.001-0.1% for benzalkonium bromide and 0.1-0.5% for tetramethylammonium chloride by weight in the PCR amplification system.

In the embodiments of the present disclosure, when the combination of benzalkonium bromide and tetramethylammonium chloride is used as the additive, the concentration range is 0.001-0.1% for benzalkonium bromide and 0.1-0.5% for tetramethylammonium chloride. When the concentrations of the two are not in such ranges, either the PCR amplification reaction efficiency or the PCR amplification specificity may be compromised. It will be appreciated that 0.001-0.1% benzalkonium bromide and 0.1-0.5% for tetramethylammonium chloride refer to the concentration of the two additives by weight, and when the concentration of benzalkonium bromide by weight is in the range of 0.001-0.1%, the concentration of tetramethylammonium chloride by weight may be any value within 0.1-0.5%. For example, when the concentration of benzalkonium bromide by weight is in the range of 0.001-0.1%, the concentration of tetramethylammonium chloride by weight may be 0.1%, 0.11%, 0.12%, 0.13%, 0.14%, 0.15%, 0.16%, 0.17%, 0.18%, 0.19%, 0.2%, 0.21%, 0.22%, 0.23%, 0.24%, 0.25%, 0.26%, 0.27%, 0.28%, 0.29%, 0.3%, 0.31%, 0.32%, 0.33%, 0.34%, 0.35%, 0.36%, 0.37%, 0.38%, 0.39%, 0.4%, 0.41%, 0.42%, 0.43%, 0.44%, 0.45%, 0.46%, 0.47%, 0.48%, 0.49%, 0.5%, or the like.

In one implementation, the additive is a combination of benzalkonium bromide and tetrapolyethylene glycol monolauryl ether. In such a condition, benzalkonium bromide and tetrapolyethylene glycol monolauryl ether can improve the PCR amplification efficiency by reducing the formation of the secondary structure of DNA molecules and reducing the influence of SDS residues on the activity of DNA polymerases in the PCR amplification process.

In one embodiment, the additive is present at concentrations of 0.001-0.1% for benzalkonium bromide and 0.01-0.2% for tetrapolyethylene glycol monolauryl ether by weight in the PCR amplification system.

In the embodiments of the present disclosure, when the combination of benzalkonium bromide and tetrapolyethylene glycol monolauryl ether is used as the additive, the improvement in the PCR amplification reaction efficiency is proportional to the concentrations. When the concentration of one additive by weight is lower, the concentration of the other one by weight can be properly increased to balance the improvement effect of the additive on the PCR amplification efficiency. When the concentrations of benzalkonium bromide and tetrapolyethylene glycol monolauryl ether are higher, non-specific amplification may be introduced in multiplex PCR amplification. Thus, in some embodiments, benzalkonium bromide at a concentration by weight of 0.001-0.1% and tetrapolyethylene glycol monolauryl ether at a concentration by weight of 0.01-0.2% are combined to achieve both PCR amplification efficiency and specific amplification of multiplex PCR.

It will be appreciated that 0.001-0.1% for benzalkonium bromide and 0.01-0.2% for tetrapolyethylene glycol monolauryl ether refer to the concentration of the two additives by weight, and when the concentration of benzalkonium bromide by weight is in the range of 0.001-0.1%, the concentration of tetrapolyethylene glycol monolauryl ether by weight may be any value within 0.01-0.2%. For example, when the concentration of benzalkonium bromide by weight is in the range of 0.001-0.1%, the concentration of tetrapolyethylene glycol monolauryl ether by weight may be 0.01%, 0.02%, 0.03%, 0.04%, 0.05%, 0.06%, 0.07%, 0.08%, 0.09%, 0.1%, 0.12%, 0.15%, 0.18%, 0.2%, or the like.

In one implementation, the additive is a combination of benzalkonium bromide, tetramethylammonium chloride, and tetrapolyethylene glycol monolauryl ether. In such a condition, benzalkonium bromide and tetrapolyethylene glycol monolauryl ether can improve the PCR amplification efficiency by reducing the formation of the secondary structure of DNA molecules and reducing the influence of SDS residues on the activity of DNA polymerases in the PCR amplification process. Tetramethylammonium chloride can improve the amplification specificity and reduce the non-specific amplification possibly caused by the increase of benzalkonium bromide and tetrapolyethylene glycol monolauryl ether contents to a certain extent. The three can balance the amplification efficiency of PCR reactions and the PCR amplification specificity via their complementary and synergistic effects, and jointly improve the amplification efficiency of PCR reactions and the PCR amplification specificity.

In one embodiment, the additive is present at concentrations of 0.001-0.1% for benzalkonium bromide, 0.1-0.5% for tetramethylammonium chloride, and 0.01-0.2% for tetrapolyethylene glycol monolauryl ether by weight in the PCR amplification system. The use of the combination of benzalkonium bromide, tetramethylammonium chloride, and tetrapolyethylene glycol monolauryl ether as the additive provides excellent amplification efficiency and PCR amplification specificity by controlling the concentrations of the three substances by weight in a PCR amplification reaction system. It will be appreciated that benzalkonium bromide at any concentration by weight in the range of 0.001-0.1% can be combined with any value in the concentration range by weight of 0.1-0.5% for tetramethylammonium chloride and any value in the concentration range by weight of 0.01-0.2% for tetrapolyethylene glycol monolauryl ether. The concentrations of the additives by weight can be selected and matched among the specific values listed above, and are not limited to the specific values listed above.

In some embodiments, the additive further includes at least one of bovine serum albumin, polyethylene glycol, or dimethyl sulfoxide.

In the embodiment of the present disclosure, the additive can be used in multiplex PCR amplification to improve the reaction efficiency and the amplification specificity of the multiplex PCR amplification.

The embodiments of the present disclosure further provide a multiplex PCR amplification kit.

The kit includes a primer. In some embodiments, the primer composition is a primer composition for amplifying a plurality of target regions in M. *tuberculosis,* so as to simultaneously, separately amplify the plurality of target regions in the M. *tuberculosis* genomic DNA by multiplex PCR amplification. In one embodiment, the primer composition includes one or more primer pairs, and the primers are selected from the nucleotide sequences set forth in SEQ ID NOs. 1-174. It will be appreciated that for each target region to be amplified, the kit includes at least one pair of amplification primers, i.e., a primer pair (including an upstream primer and a downstream primer for the target region), for amplifying the target region. It means that the kit includes an even number of primers to form one or more primer pairs, and the number of primer pairs corresponds to the number of the target regions to be amplified.

In this embodiment, the primers are selected from the nucleotide sequences set forth in SEQ ID NOs. 1-174, and refer to primer pairs for one or more target regions to be amplified in the nucleotide sequences set forth in SEQ ID NOs. 1-174. In one embodiment, the primer composition is a nucleotide sequence set forth in SEQ ID NOs. 1-174. In such a condition, the amplification primers are adapted to the NGS sequencing length, covering, for example, multiple resistance loci in M. *tuberculosis* for resistance to multiple drugs, so as to achieve the specific amplification of all relevant resistance loci in M. *tuberculosis* and further analyze the drug resistance thereof. As such, the analysis of multiple drug resistance loci can be achieved in one run. In some embodiments, such drugs include rifampicin, isoniazid, ethambutol, streptomycin, fluoroquinolone, ethionamide, p-aminosalicylic acid, clofazimine, D-cycloserine, kanamycin, amikacin, levofloxacin, moxifloxacin, bedaquiline, linezolid, capreomycin, delamanid, and pyrazinamide, and the drug-resistance genes encompass rpoB for rifampicin, fabG1/inhA/katG for isoniazid, embB/embA for ethambutol, rpsL/gid/rrs for streptomycin, gyrA/gyrB for fluoroquinolone, ethA/fabG1/inhA for ethionamide, folC/ribD/thyA for p-aminosalicylic acid, Rv0678 for clofazimine, Alr/pncA/ald for D-cycloserine, eis/rrs for kanamycin, rrs for amikacin, rrs/tlyA for levofloxacin, ddn/fbiA/rplC/rrl for moxifloxacin, Rv0678 for bedaquiline, rplC for linezolid, gyrA/gyrB for capreomycin, gyrA/gyrB for delamanid, and pncA for pyrazinamide. That is, the nucleotide sequences set forth in SEQ ID NOs. 1-174 can achieve the specific amplification of part or all of the drug resistance loci in one step, and are helpful to synchronously achieving the analysis of multidrug resistance.

In the embodiments of the present disclosure, the kit further includes a DNA polymerase and dNTPs. The type of DNA polymerase is not strictly limited, and existing common DNA polymerases can be selected. dNTPs are substrates for the amplification reaction, and include dATP, dCTP, dGTP, and dTTP.

In the embodiments of the present disclosure, the kit further includes a PCR amplification reagent. In a kit for amplifying target regions in M. *tuberculosis,* the PCR amplification reagent includes but is not limited to KCI, MgCl₂, and Tris-HCl.

In some embodiments, the PCR amplification reagent includes an additive. In one embodiment, the additive is the additive containing benzalkonium bromide described above.

In some embodiments, benzalkonium bromide is present at a concentration of 0.001-0.1% by weight in a PCR amplification system. The effect of the concentration of benzalkonium bromide by weight on PCR amplification is as described above and will not be recited for brevity.

In some embodiments, the additive further includes tetramethylammonium chloride and/or tetrapolyethylene glycol monolauryl ether. In one implementation, the additive is a combination of benzalkonium bromide and tetramethylammonium chloride. In such a condition, the additive may be present at concentrations of 0.001-0.1% for benzalkonium bromide and 0.1-0.5% for tetramethylammonium chloride by weight in the PCR amplification system. In one implementation, the additive is a combination of benzalkonium bromide and tetrapolyethylene glycol monolauryl ether. In such a condition, the additive is present at concentrations of 0.001-0.1% for benzalkonium bromide and 0.01-0.2% for tetrapolyethylene glycol monolauryl ether by weight in the PCR amplification system. The additive is present at concentrations of 0.001-0.1% for benzalkonium bromide, 0.1-0.5% for tetramethylammonium chloride, and 0.01-0.2% for tetrapolyethylene glycol monolauryl ether by weight in the PCR amplification system. In such a condition, the additive is present at concentrations of 0.001-0.1% for benzalkonium bromide, 0.1-0.5% for tetramethylammonium chloride, and 0.01-0.2% for tetrapolyethylene glycol monolauryl ether by weight in the PCR amplification system. The additive combinations and the effects of their concentrations by weight in various implementations are as described above and will not be recited for brevity.

In a third aspect, the embodiments of the present disclosure provide use of a multiplex PCR amplification kit in drug resistance detection in M. *tuberculosis,* where the multiplex PCR amplification kit is as described above and is not recited.

In a fourth aspect, the embodiments of the present disclosure further provide a method for detecting drug resistance in M. *tuberculosis,* including:
performing multiplex PCR amplification on a M. *tuberculosis* culture using the multiplex PCR amplification kit according to the second aspect; sequencing the amplification product; and determining the drug resistance of M. *tuberculosis* based on the sequencing result.

The embodiments of the present disclosure achieve the drug-resistance gene detection in M. *tuberculosis* by using a multiplex PCR targeted capture technique and a high-throughput second-generation sequencing technique, where the multiplex PCR amplification reagent has an important influence on the amplification effect. The present disclosure utilizes an additive for promoting the amplification in the PCR amplification process, which can increase the amplification efficiency and specificity and has an important effect on the subsequent library construction and analysis.

The present application will be illustrated with reference to the following specific examples.

### Example 1

This example provides a PCR amplification method including the following procedures:
(1) Preparation of template: Human blood was collected in an EDTA (ethylene diamine tetraacetic acid) anticoagulation tube. 200 µL of blood was transferred in a 1.5-mL centrifuge tube after the blood sample was well mixed to extract the DNA in the blood sample.
(2) Specific primers designed for the ACTB gene were provided with the sequence of the primers shown in Table 1. In the table, "name-F" or "name-R" denotes the name of the primers, where the "name" denotes the name of the nucleic acid template. In the following table, the primers are named after the loci of interest, where F denotes the upstream primer, and R denotes the downstream primer.

**Table 1**

| Serial number | Primer | Sequence number | Primer sequence (5'-3') |
|---|---|---|---|
| 1 | ACTB-F | SEQ ID NO:175 | GCTCAGGGCTTCTTGTCCTT |
| 2 | ACTB-R | SEQ ID NO:176 | TCGATGGGGTACTTCAGGGT |

(3) PCR reaction systems were prepared according to the formulas in Table 2, where the additive was BKB (benzalkonium bromide) at a concentration by weight of 0.1% in the PCR reaction system. Three groups of PCR reaction systems containing templates at different concentrations were prepared, where the nucleic acid template concentrations were 0.1 ng, 10 ng, and 100 ng, respectively.

**Table 2**

| Components | 1× final concentration |
|---|---|
| KCl | 600 mM |
| MgCl₂ | 3 mM |
| Tris-HCl PH=8.4 | 300 mM |
| dNTP | 0.2 mM |
| Primers mix | 0.1 µM |
| OneTaq^{®} Hot Start DNA Polymerase(5U/µL) | 0.05 U/µL |
| BKB (benzalkonium bromide) | 0.1% |
| Nucleic acid template | 0.1 ng/10 ng/100 ng |
| ddH₂O | Dilution to 25 µL |

PCR amplification procedures: Pre-denaturation at 95 °C for 2 min; ×30 cycles (denaturation at 98 °C for 20 s; annealing at 60 °C for 20 s; extension at 72 °C for 30 s); final extension at 72 °C for 3 min; incubation at 4 °C.

### Example 2

This example provides a PCR amplification method different from that in Example 1 in that the concentration of BKB (benzalkonium bromide) by weight in the PCR reaction system in step (3) was 0.01%, while the rest of the steps are identical to Example 1.

### Example 3

This example provides a PCR amplification method different from that in Example 1 in that the concentration of BKB (benzalkonium bromide) by weight in the PCR reaction system in step (3) was 0.001%, while the rest of the steps are identical to Example 1.

### Comparative Example 1

This comparative example provides a PCR amplification method different from that in Example 1 in that the additive was BSA (bovine serum albumin) at a concentration by weight of 5% in the PCR reaction system in step (3), while the rest of the steps are identical to Example 1.

### Comparative Example 2

This comparative example provides a PCR amplification method different from that in Example 1 in that the additive was BSA (bovine serum albumin) at a concentration by weight of 1% in the PCR reaction system in step (3), while the rest of the steps are identical to Example 1.

### Comparative Example 3

This comparative example provides a PCR amplification method different from that in Example 1 in that the additive was BSA (bovine serum albumin) at a concentration by weight of 8% in the PCR reaction system in step (3), while the rest of the steps are identical to Example 1.

### Comparative Example 4

This comparative example provides a PCR amplification method different from that in Example 1 in that the additive was PEG8000 (polyethylene glycol) at a concentration by weight of 0.1% in the PCR reaction system in step (3), while the rest of the steps are identical to Example 1.

### Comparative Example 5

This comparative example provides a PCR amplification method different from that in Example 1 in that the additive was PEG8000 (polyethylene glycol) at a concentration by weight of 0.5% in the PCR reaction system in step (3), while the rest of the steps are identical to Example 1.

### Comparative Example 6

This comparative example provides a PCR amplification method different from that in Example 1 in that the additive was PEG8000 (polyethylene glycol) at a concentration by weight of 0.05% in the PCR reaction system in step (3), while the rest of the steps are identical to Example 1.

### Comparative Example 7

This comparative example provides a PCR amplification method different from that in Example 1 in that no extra additives were added in step (3), while the rest of the steps are identical to Example 1.

The amplification products obtained by the PCR amplification methods provided in Examples 1-3 and Comparative Examples 1-7 were subjected to agarose gel electrophoresis. The amplification products obtained in Examples 1-3 and Comparative Examples 1-6 were analyzed using grayscale analysis software based on the electrophoretogram of Comparative Example 7. The amplification efficiency and amplification specificity analysis results for the PCR amplification methods in Examples 1-3 and Comparative Examples 1-7 are shown in Table 3.

**Table 3**

| Serial number | Final concentration of template | Additive | Final concentration | Simplex PCR reaction | |
|---|---|---|---|---|---|
| | | | | Amplification efficiency | Amplification specificity |
| Example 1 | 0.1 ng | BKB (benzalkonium bromide) | 0.1% | (++) | (++) |
| | 10 ng | | | (++) | (+) |
| | 100 ng | | | (++) | (+) |
| Example 2 | 0.1 ng | | 0.01% | (+++) | (++) |
| | 10 ng | | | (+++) | (++) |
| | 100 ng | | | (+++) | (++) |
| Example 3 | 0.1 ng | | 0.001% | (+) | (++) |
| | 10 ng | | | (+) | (++) |
| | 100 ng | | | (+) | (+) |
| Comparative Example 1 | 0.1 ng | BSA (bovine serum albumin) | 5% | (/) | (/) |
| | 10 ng | | | (/) | (/) |
| | 100 ng | | | (/) | (/) |
| Comparative Example 2 | 0.1 ng | | 1% | (/) | (/) |
| | 10 ng | | | (/) | (/) |
| | 100 ng | | | (/) | (/) |
| Comparative Example 3 | 0.1 ng | | 8% | (+) | (/) |
| | 10 ng | | | (+) | (/) |
| | 100 ng | | | (/) | (/) |
| Comparative Example 4 | 0.1 ng | PEG8000 (polyethylene glycol) | 0.1% | (+) | (-) |
| | 10 ng | | | (+) | (-) |
| | 100 ng | | | (+) | (-) |
| Comparative Example 5 | 0.1 ng | | 0.5% | (-) | (-) |
| | 10 ng | | | (-) | (-) |
| | 100 ng | | | (-) | (-) |
| Comparative Example 6 | 0.1 ng | | 0.05% | (/) | (+) |
| | 10 ng | | | (/) | (+) |
| | 100 ng | | | (/) | (/) |
| Comparative Example 7 | 0.1 ng | No additives | / | (/) | (/) |
| | 10 ng | | / | (/) | (/) |
| | 100 ng | | / | (/) | (/) |

A higher PCR amplification product content and a lower non-specific fragment content indicate a higher amplification efficiency, since the generation of non-specific fragments can reduce the content of target fragments and thus lead to poor amplification efficiency and specificity. In the examples of the present application, the amplification efficiency and the amplification specificity of the PCR amplification products were assessed by detecting the grayscale values and the number of non-target bands in an agarose gel electrophoretogram. Particularly, a higher grayscale value in the agarose gel electrophoretogram indicates a greater amount of amplification product; a single band at the position of the target fragment indicates good specificity, the presence of bands at the positions of non-target fragments indicates poor specificity, and the brightness and the width of the target band indicate high amplification yield and high amplification efficiency.

According to the above table, in the amplification efficiency test, "-" denotes a target band with a reduced grayscale value or no target band in the agarose gel electrophoretogram as compared with the agarose gel electrophoresis result of the blank control (Comparative Example 7); "/" denotes essentially no effects; "+" denotes a 30% increase in the grayscale value of the target band in the agarose gel electrophoretogram as compared with the agarose gel electrophoresis results of the blank control (Comparative Example 7), and the number of "+" denotes the degree of increase, where a greater number of plus signs indicates a more distinct contrast from the blank control (Comparative Example 7).

In the amplification specificity test, "-" denotes more non-target bands or elevated grayscale values in the agarose gel electrophoretogram as compared with the agarose gel electrophoresis result of the blank control (Comparative Example 7); "/" denotes essentially no effects; "+" denotes a 10% decrease in the grayscale value of the non-target bands in the agarose gel electrophoretogram as compared with the agarose gel electrophoresis results of the blank control (Comparative Example 7), and the number of "+" denotes the degree of decrease of the non-target bands, where a greater number of plus signs indicates a more distinct contrast from the blank control (Comparative Example 7). It will be appreciated that the assessment criteria are also applicable to the assessment of the amplification effects of the following examples, comparative examples, and blank controls.

As can be seen from Table 3, BKB (benzalkonium bromide) exhibited good amplification effects at a concentration by weight of 0.001-0.1%, and the slight decrease in amplification efficiency at a BKB concentration by weight of 0.1% may be attributed to the decrease in amplification specificity; BSA and PEG8000, when used alone, exhibited poor amplification-promoting effects. It can be seen that BKB (benzalkonium bromide), when used as an additive, has a good amplification-promoting effect at nucleic acid template amounts of 0.1 ng, 10 ng, and 100 ng. In addition, at a low nucleic acid template amount, the promoting effects of BKB (benzalkonium bromide) on amplification efficiency and amplification specificity are more significant than those of the comparative example with no additives.

### Example 4

This example provides a multiplex PCR method including the following procedures:
(1) 5 mL of M. *tuberculosis* culture was centrifuged 3 times and collected into a 1.5-mL centrifuge tube to give a M. *tuberculosis* sample. DNA was extracted from the M. *tuberculosis* sample.
(2) 13 primer pairs as shown in Table 4 below were provided. In the table, "name-F" or "name-R" denotes the name of the primers, where the "name" denotes the name of the nucleic acid template. In the following table, the primers are named after the loci of interest, where F denotes the upstream primer, and R denotes the downstream primer. The amplification primers for the same nucleic acid template included an upstream primer and a downstream primer to form the primer pair. For example, "katG-F" and "katG-R" refer to the upstream and downstream primers, respectively, for amplifying the same nucleic acid template containing katG locus, and "katG-F" and "katG-R" consist of the primer pair that amplifies the nucleic acid template.

**Table 4**

| Serial number | Primer | Sequence number | Serial number | Primer | Sequence number |
|---|---|---|---|---|---|
| 1 | katG-F | SEQ ID NO:1 | 14 | katG-R | SEQ ID NO:14 |
| 2 | inhA-F | SEQ ID NO:2 | 15 | inhA-R | SEQ ID NO:15 |
| 3 | rpoB-F | SEQ ID NO:3 | 16 | rpoB-R | SEQ ID NO:16 |
| 4 | embB-F | SEQ ID NO:4 | 17 | embB-R | SEQ ID NO:17 |
| 5 | gyrA-F | SEQ ID NO:5 | 18 | gyrA-R | SEQ ID NO:18 |
| 6 | 16S-F | SEQ ID NO:6 | 19 | 16S-R | SEQ ID NO:19 |
| 7 | atpE-F | SEQ ID NO:7 | 20 | atpE-R | SEQ ID NO:20 |
| 8 | murA-F | SEQ ID NO:8 | 21 | murA-R | SEQ ID NO:21 |
| 9 | rpsL-F | SEQ ID NO:9 | 22 | rpsL-R | SEQ ID NO:22 |
| 10 | rpsA-F | SEQ ID NO:10 | 23 | rpsA-R | SEQ ID NO:23 |
| 11 | ribD-2-F | SEQ ID NO:11 | 24 | ribD-2-R | SEQ ID NO:24 |
| 12 | IS6110-F | SEQ ID NO:12 | 25 | IS6110-R | SEQ ID NO:25 |
| 13 | M.bovis-F | SEQ ID NO:13 | 26 | M.bovis-R | SEQ ID NO:26 |

(3) Referring to Table 2, the PCR reaction system was prepared for multiplex PCR amplification with 0.1 ng of template, where the additive used was a combination of TMAC and BKB with concentrations by weight of 0.3% for TMAC and 0.01% for BKB in the PCR reaction system.

The multiplex PCR amplification procedures were: incubation at 95 °C for 2 min; 30 cycles of incubation at 98 °C for 20 s, incubation at 65 °C for 1 min, and incubation at 72 °C for 30 s; incubation at 72 °C for 3 min; and incubation at 4 °C. After the amplification, the amplification efficiency and the amplification specificity were measured in accordance with Example 1.

### Example 5

This example provides a multiplex PCR method different from that in Example 4 in that the additive was a combination of BSA and BKB at concentrations by weight of 8% for BSA and 0.01% for BKB in the PCR reaction system in step (3), while the rest of the steps are identical to Example 4.

### Example 6

This example provides a multiplex PCR method different from that in Example 4 in that the additive was a combination of PEG and BKB at concentrations by weight of 0.1% for PEG and 0.01% for BKB in the PCR reaction system in step (3), while the rest of the steps are identical to Example 4.

### Example 7

This example provides a multiplex PCR method different from that in Example 4 in that the additive was a combination of DMSO and BKB at concentrations by weight of 0.5% for DMSO and 0.01% for BKB in the PCR reaction system in step (3), while the rest of the steps are identical to Example 4.

### Example 8

This example provides a multiplex PCR method different from that in Example 4 in that the additive was a combination of Theist and BKB at concentrations by weight of 0.2% for Theist and 0.01% for BKB in the PCR reaction system in step (3), while the rest of the steps are identical to Example 4.

### Comparative Example 8

This comparative example provides a multiplex PCR method different from that in Example 4 in that the additive was a combination of PEG and Theist at concentrations by weight of 0.1% for PEG and 0.2% for Theist in the PCR reaction system in step (3), while the rest of the steps are identical to Example 4.

### Comparative Example 9

This comparative example provides a multiplex PCR method different from that in Example 4 in that the additive was a combination of DMSO and Theist at concentrations by weight of 0.5% for DMSO and 0.2% for Theist in the PCR reaction system in step (3), while the rest of the steps are identical to Example 4.

The amplification efficiency and amplification specificity analysis results for the PCR amplification methods in Examples 4-8 and Comparative Examples 8 and 9 are shown in Table 5.

**Table 5**

| Group | Additive combination | Amplification efficiency | Amplification specificity |
|---|---|---|---|
| Example 4 | 0.3% TMAC+0.01% BKB | (+++) | (+++) |
| Example 5 | 8% BSA+0.01% BKB | (++) | (+) |
| Example 6 | 0.1% PEG+0.01% BKB | (+) | (+) |
| Example 7 | 0.5% DMSO+0.01% BKB | (++) | (++) |
| Example 8 | 0.2%Theist+0.01% BKB | (+++) | (+) |
| Comparative Example 8 | 0.1% PEG+0.2% Theist | (+) | (-) |
| Comparative Example 9 | 0.5% DMSO+0.2% Theist | (+) | (-) |
| 13-plex PCR blank control | No additives | (/) | (/) |

The results were compared with a control group with no additives (a 13-plex PCR blank control prepared according to Example 4 but different from Example 4 in that no additives were used). As can be seen from Table 5, the additive combinations of Examples 4, 5, 7, and 8 exhibited promoting effects on both amplification efficiency and amplification specificity, where the promoting effects of the additive combinations of Examples 4 and 8 are particularly significant.

### Example 9

This example provides a multiplex PCR method different from that in Example 4 in that the concentrations by weight of TMAC and BKB were, respectively, 0.1% for TMAC and 0.1% for BKB in the PCR reaction system in step (3). The rest of the steps are identical to Example 4.

### Example 10

This example provides a multiplex PCR method different from that in Example 4 in that the concentrations by weight of TMAC and BKB were, respectively, 0.5% for TMAC and 0.001% for BKB in the PCR reaction system in step (3). The rest of the steps are identical to Example 4.

### Example 11

This example provides a multiplex PCR method different from that in Example 8 in that the additive was a combination of Theist and BKB at concentrations by weight of 0.001% for Theist and 0.1% for BKB in the PCR reaction system in step (3). The rest of the steps are identical to Example 8.

### Example 12

This example provides a multiplex PCR method different from that in Example 8 in that the additive was a combination of Theist and BKB at concentrations by weight of 0.1% for Theist and 0.001% for BKB in the PCR reaction system in step (3). The rest of the steps are identical to Example 8.

The amplification efficiency and amplification specificity analysis results for the PCR amplification methods in Examples 9-12 are shown in Table 6.

**Table 6**

| Group | Additive combination | Amplification efficiency | Amplification specificity |
|---|---|---|---|
| Example 9 | 0.1% TMAC+0.1% BKB | (+++) | (+++) |
| Example 10 | 0.5% TMAC+0.001% BKB | (+++) | (+++) |
| Example 11 | 0.001% Theist+0.1% BKB | (+++) | (++) |
| Example 12 | 0.1% Theist+0.001% BKB | (++) | (++) |
| 13-plex PCR blank control | No additives | 0 | 0 |

As shown in Table 6, the combination of TMAC and BKB as an additive at a TMAC concentration of 0.1-0.5% also exhibited excellent amplification effects. The combination of Theist and BKB as an additive at a Theist concentration of 0.001-0.2% exhibited excellent amplification effects.

### Example 13

This example provides a multiplex PCR method including the following procedures:
(1) 5 mL of M. *tuberculosis* culture was centrifuged 3 times and collected into a 1.5-mL centrifuge tube to give a M. *tuberculosis* sample. DNA was extracted from the M. *tuberculosis* sample.
(2) 13 primer pairs as shown in Table 4 were provided for 13-plex PCR amplification.
(3) Referring to Table 2, the PCR reaction system was prepared for multiplex PCR amplification with 0.1 ng of template, where the additive used was a combination of TMAC, BKB, and DMSO with concentrations by weight of 0.3% for TMAC, 0.01% for BKB, and 0.5% for DMSO in the PCR reaction system.

The multiplex PCR amplification procedures were: incubation at 95 °C for 2 min; 30 cycles of incubation at 98 °C for 20 s, incubation at 65 °C for 1 min, and incubation at 72 °C for 30 s; incubation at 72 °C for 3 min; and incubation at 4 °C. After the amplification, the amplification efficiency and the amplification specificity were measured in accordance with Example 1.

### Example 14

This example provides a multiplex PCR method different from that in Example 13 in that the additive was a combination of TMAC, BKB, and Theist at concentrations by weight of 0.3% for TMAC, 0.01% for BKB, and 0.2% for Theist in the PCR reaction system in step (3), while the rest of the steps are identical to Example 13.

### Comparative Example 10

This comparative example provides a multiplex PCR method different from that in Example 13 in that the additive was a combination of TMAC, Theist, and BSA at concentrations by weight of 0.3% for TMAC, 0.2% for Theist, and 8% for BSA in the PCR reaction system in step (3), while the rest of the steps are identical to Example 13.

### Comparative Example 11

This comparative example provides a multiplex PCR method different from that in Example 13 in that the additive was a combination of TMAC, Theist, and PEG at concentrations by weight of 0.3% for TMAC, 0.2% for Theist, and 0.1% for PEG in the PCR reaction system in step (3), while the rest of the steps are identical to Example 13.

The amplification efficiency and amplification specificity analysis results for the PCR amplification methods in Examples 13 and 14 and Comparative Examples 10 and 11 are shown in Table 7.

**Table 7**

| Group | Additive combination | Amplification efficiency | Amplification specificity |
|---|---|---|---|
| Example 13 | 0.3% TMAC+0.01% BKB+0.5% DMSO | (++) | (++) |
| Example 14 | 0.3% TMAC+0.01% BKB+0.2% Theist | (+++++) | (+++++) |
| Comparative Example 10 | 0.3% TMAC+0.2% Theist+8% BSA | (++) | (++) |
| Comparative Example 11 | 0.3% TMAC+0.2% Theist+0.1% PEG | (+) | (+) |
| 13-plex PCR blank control | No additives | 0 | 0 |

As can be seen from Table 7, the additive combination of TMAC, BKB, and Theist exhibited superior amplification effects, with higher amplification efficiency and amplification specificity. In addition, when using an additive containing TMAC, BKB, and Theist in PCR amplification, the amount of additive can be reduced.

### Example 15

This example provides a multiplex PCR method different from that in Example 13 in that the additive was a combination of TMAC, BKB, and Theist at concentrations by weight of 0.5% for TMAC, 0.001% for BKB, and 0.01% for Theist in the PCR reaction system in step (3), while the rest of the steps are identical to Example 13.

### Example 16

This example provides a multiplex PCR method different from that in Example 13 in that the additive was a combination of TMAC, BKB, and Theist at concentrations by weight of 0.1% for TMAC, 0.1% for BKB, and 0.01% for Theist in the PCR reaction system in step (3), while the rest of the steps are identical to Example 13.

### Example 17

This example provides an additive for multiplex PCR amplification by a method different from that in Example 13 in that the additive was a combination of TMAC, BKB, and Theist at concentrations by weight of 0.1% for TMAC, 0.0005% for BKB, and 0.001% for Theist in the PCR reaction system, while the rest of the steps were identical to Example 13.

### Example 18

This example provides an additive for multiplex PCR amplification by a method different from that in Example 13 in that the additive was a combination of TMAC, BKB, and Theist at concentrations by weight of 0.1% for TMAC, 0.2% for BKB, and 0.001% for Theist in the PCR reaction system, while the rest of the steps are identical to Example 13.

The amplification efficiency and amplification specificity analysis results for the PCR amplification methods in Examples 15-18 are shown in Table 8.

**Table 8**

| Group | Additive combination | Amplification efficiency | Amplification specificity |
|---|---|---|---|
| 13-plex PCR blank control | No additives | 0 | 0 |
| Example 14 | 0.3% TMAC+0.01% BKB+0.2% Theist | (+++++) | (+++++) |
| Example 15 | 0.5% TMAC+0.001% BKB+0.01% Theist | (++++) | (++++) |
| Example 16 | 0.1% TMAC+0.1% BKB+0.01% Theist | (+++) | (++) |
| Example 17 | 0.1% TMAC+0.0005% BKB+0.001% Theist | (+) | (+) |
| Example 18 | 0.1% TMAC+0.2% BKB+0.001%Theist | (+) | (+) |

As can be seen from Table 8, Examples 14, 15, and 16 exhibited good amplification effects, with high amplification efficiency and good amplification specificity. As can be seen from the comparison of Examples 14, 17, and 18, the promoting effects on amplification efficiency and amplification specificity in multiplex PCR amplification were reduced when the concentration by weight of benzalkonium bromide in the additive combination was out of the range of 0.001-0.1%. In the examples of the present disclosure, benzalkonium bromide, tetramethylammonium chloride, and tetrapolyethylene glycol monolauryl ether can cooperate with each other, and the amplification effects of multiplex PCR amplification can be maximized when the concentrations of the three are controlled at proper levels.

### Example 19

This example provides a multiplex PCR method including the following procedures:
(1) 5 mL of M. *tuberculosis* culture was centrifuged 3 times and collected into a 1.5-mL centrifuge tube to give a M. *tuberculosis* sample. DNA was extracted from the M. *tuberculosis* sample.
(2) 74 primer pairs, as shown in Table 9, were provided. In the table, "name-F" or "name-R" denotes the name of the primers, where the "name" denotes the name of the nucleic acid template. In the following table, the primers are named after the loci of interest, where F denotes the upstream primer, and R denotes the downstream primer. The amplification primers for the same nucleic acid template included an upstream primer and a downstream primer to form the primer pair. The "pncA-1-F, pncA-1-R, pncA-2-F, pncA-2-R, pncA-3-F, pncA-3-R, pncA-4-F, and pncA-4-R" denote upstream and downstream primers for the four divided fragments of pncA, and other primers are named similarly.

**Table 9**

| Serial number | Name | Sequence number | Serial number | Name | Sequence number | Serial number | Name | Sequence number |
|---|---|---|---|---|---|---|---|---|
| 1 | pncA-1-F | SEQ ID NO:27 | 51 | katG-7-F | SEQ ID NO:77 | 101 | ethA-2-F | SEQ ID NO:127 |
| 2 | pncA-1-R | SEQ ID NO:28 | 52 | katG-7-R | SEQ ID NO:78 | 102 | ethA-2-R | SEQ ID NO:128 |
| 3 | pncA-2-F | SEQ ID NO:29 | 53 | katG-8-F | SEQ ID NO:79 | 103 | ethA-3-F | SEQ ID NO:129 |
| 4 | pncA-2-R | SEQ ID NO:30 | 54 | katG-8-R | SEQ ID NO:80 | 104 | ethA-3-R | SEQ ID NO:130 |
| 5 | pncA-3-F | SEQ ID NO:31 | 55 | katG-9-F | SEQ ID NO:81 | 105 | ethA-4-F | SEQ ID NO:131 |
| 6 | pncA-3-R | SEQ ID NO:32 | 56 | katG-9-R | SEQ ID NO:82 | 106 | ethA-4-R | SEQ ID NO:132 |
| 7 | pncA-4-F | SEQ ID NO:33 | 57 | katG-10-F | SEQ ID NO:83 | 107 | ethA-5-F | SEQ ID NO:133 |
| 8 | pncA-4-R | SEQ ID NO:34 | 58 | katG-10-R | SEQ ID NO:84 | 108 | ethA-5-R | SEQ ID NO:134 |
| 9 | gyrA-F | SEQ ID NO:35 | 59 | embA-F | SEQ ID NO:85 | 109 | ethA-6-F | SEQ ID NO:135 |
| 10 | gyrA-R | SEQ ID NO:36 | 60 | embA-R | SEQ ID NO:86 | 110 | ethA-6-R | SEQ ID NO:136 |
| 11 | embB-1-F | SEQ ID NO:37 | 61 | Rv0678-3-F | SEQ ID NO:87 | 111 | eis-2-F | SEQ ID NO:137 |
| 12 | embB-1-R | SEQ ID NO:38 | 62 | Rv0678-3-R | SEQ ID NO:88 | 112 | eis-2-R | SEQ ID NO:138 |
| 13 | embB-2-F | SEQ ID NO:39 | 63 | Rv0678-4-F | SEQ ID NO:89 | 113 | ethA-7-F | SEQ ID NO:139 |
| 14 | embB-2-R | SEQ ID NO:40 | 64 | Rv0678-4-R | SEQ ID NO:90 | 114 | ethA-7-R | SEQ ID NO:140 |
| 15 | embB-3-F | SEQ ID NO:41 | 65 | foIC-1-F | SEQ ID NO:91 | 115 | ethA-8-F | SEQ ID NO:141 |
| 16 | embB-3-R | SEQ ID NO:42 | 66 | folC-1-R | SEQ ID NO:92 | 116 | ethA-8-R | SEQ ID NO:142 |
| 17 | embB-4-F | SEQ ID NO:43 | 67 | folC-2-F | SEQ ID NO:93 | 117 | gid-4-F | SEQ ID NO:143 |
| 18 | embB-4-R | SEQ ID NO:44 | 68 | folC-2-R | SEQ ID NO:94 | 118 | gid-4-R | SEQ ID NO:144 |
| 19 | embB-5-F | SEQ ID NO:45 | 69 | folC-3-F | SEQ ID NO:95 | 119 | ald-F | SEQ ID NO:145 |
| 20 | embB-5-R | SEQ ID NO:46 | 70 | folC-3-R | SEQ ID NO:96 | 120 | ald-R | SEQ ID NO:146 |
| 21 | alr-1-F | SEQ ID NO:47 | 71 | rpoB-1-F | SEQ ID NO:97 | 121 | eis-F | SEQ ID NO:147 |
| 22 | alr-1-R | SEQ ID NO:48 | 72 | rpoB-1-R | SEQ ID NO:98 | 122 | eis-R | SEQ ID NO:148 |
| 23 | alr-2-F | SEQ ID NO:49 | 73 | rpoB-2-F | SEQ ID NO:99 | 123 | rrs-1-F | SEQ ID NO:149 |
| 24 | alr-2-R | SEQ ID NO:50 | 74 | rpoB-2-R | SEQ ID NO:100 | 124 | rrs-1-R | SEQ ID NO:150 |
| 25 | alr-3-F | SEQ ID NO:51 | 75 | inhA-F | SEQ ID NO:101 | 125 | rrs-2-F | SEQ ID NO:151 |
| 26 | alr-3-R | SEQ ID NO:52 | 76 | inhA-R | SEQ ID NO:102 | 126 | rrs-2-R | SEQ ID NO:152 |
| 27 | gid-1-F | SEQ ID NO:53 | 77 | rplC-F | SEQ ID NO:103 | 127 | rrs-3-F | SEQ ID NO:153 |
| 28 | gid-1-R | SEQ ID NO:54 | 78 | rpIC-R | SEQ ID NO:104 | 128 | rrs-3-R | SEQ ID NO:154 |
| 29 | gid-2-F | SEQ ID NO:55 | 79 | rpsL-1-F | SEQ ID NO:105 | 129 | ribD-F | SEQ ID NO:155 |
| 30 | gid-2-R | SEQ ID NO:56 | 80 | rpsL-1-R | SEQ ID NO:106 | 130 | ribD-R | SEQ ID NO:156 |
| 31 | gid-3-F | SEQ ID NO:57 | 81 | tlyA-1-F | SEQ ID NO:107 | 131 | katG-1-F | SEQ ID NO:157 |
| 32 | gid-3-R | SEQ ID NO:58 | 82 | tlyA-1-R | SEQ ID NO:108 | 132 | katG-1-R | SEQ ID NO:158 |
| 33 | fbiA-F | SEQ ID NO:59 | 83 | tlyA-2-F | SEQ ID NO:109 | 133 | katG-2-F | SEQ ID NO:159 |
| 34 | fbiA-R | SEQ ID NO:60 | 84 | tlyA-2-R | SEQ ID NO:110 | 134 | katG-2-R | SEQ ID NO:160 |
| 35 | thyA-1-F | SEQ ID NO:61 | 85 | tlyA-3-F | SEQ ID NO:111 | 135 | katG-4-F | SEQ ID NO:161 |
| 36 | thyA-1-R | SEQ ID NO:62 | 86 | tlyA-3-R | SEQ ID NO:112 | 136 | katG-4-R | SEQ ID NO:162 |
| 37 | thyA-2-F | SEQ ID NO:63 | 87 | tlyA-4-F | SEQ ID NO:113 | 137 | Rv0678-2-F | SEQ ID NO:163 |
| 38 | thyA-2-R | SEQ ID NO:64 | 88 | tlyA-4-R | SEQ ID NO:114 | 138 | Rv0678-2-R | SEQ ID NO:164 |
| 39 | thyA-3-F | SEQ ID NO:65 | 89 | tlyA-5-F | SEQ ID NO:115 | 139 | rpoB-3-F | SEQ ID NO:165 |
| 40 | thyA-3-R | SEQ ID NO:66 | 90 | tlyA-5-R | SEQ ID NO:116 | 140 | rpoB-3-R | SEQ ID NO:166 |
| 41 | thyA-4-F | SEQ ID NO:67 | 91 | fabG1-1-F | SEQ ID NO:117 | 141 | rpsL-F | SEQ ID NO:167 |
| 42 | thyA-4-R | SEQ ID NO:68 | 92 | fabG1-1-R | SEQ ID NO:118 | 142 | rpsL-R | SEQ ID NO:168 |
| 43 | thyA-5-F | SEQ ID NO:69 | 93 | fa bG 1-2-F | SEQ ID NO:119 | 143 | rrl-1-F | SEQ ID NO:169 |
| 44 | thyA-5-R | SEQ ID NO:70 | 94 | fabG1-2-R | SEQ ID NO:120 | 144 | rrl-1-R | SEQ ID NO:170 |
| 45 | katG-3-F | SEQ ID NO:71 | 95 | ddn-F | SEQ ID NO:121 | 145 | ethA-1-F | SEQ ID NO:171 |
| 46 | katG-3-R | SEQ ID NO:72 | 96 | ddn-R | SEQ ID NO:122 | 146 | ethA-1-R | SEQ ID NO:172 |
| 47 | katG-5-F | SEQ ID NO:73 | 97 | gyrB-1-F | SEQ ID NO:123 | 147 | rpoB-3-F | SEQ ID NO:173 |
| 48 | katG-5-R | SEQ ID NO:74 | 98 | gyrB-1-R | SEQ ID NO:124 | 148 | rpoB-3-R | SEQ ID NO:174 |
| 49 | katG-6-F | SEQ ID NO:75 | 99 | gyrB-2-F | SEQ ID NO:125 | 149 | / | / |
| 50 | katG-6-R | SEQ ID NO:76 | 100 | gyrB-2-R | SEQ ID NO:126 | 150 | / | / |

(3) Referring to Table 2, the PCR reaction system was prepared for multiplex PCR amplification with 0.1 ng of template, where the additive used was a combination of TMAC, BKB, and Theist with concentrations by weight of 0.3% for TMAC, 0.01% for BKB, and 0.2% for Theist in the PCR reaction system.

The multiplex PCR amplification procedures were: incubation at 95 °C for 2 min; 30 cycles of incubation at 98 °C for 20 s, incubation at 65 °C for 1 min, and incubation at 72 °C for 30 s; incubation at 72 °C for 3 min; and incubation at 4 °C. After the amplification, the amplification efficiency and the amplification specificity were measured in accordance with Example 1.

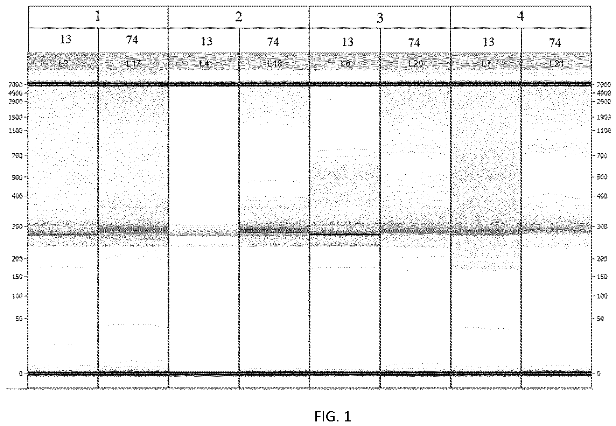

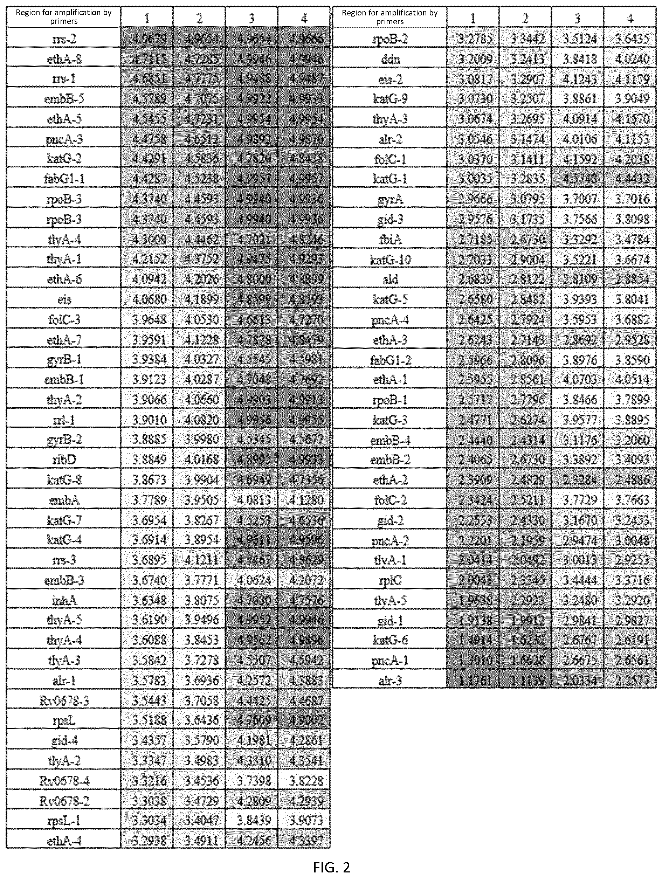

FIG. 1 illustrates the electrophoretogram of amplified libraries obtained by 13-plex or 74-plex PCR amplification. Set 1 shows the electrophoretogram of the chip after 13-plex and 74-plex amplifications and library construction when the composition (including concentration by weight) of the additive combination was 0.3% of TMAC, 0.01% of BKB and 0.2% of Theist and the nucleic acid template was 0.1 ng of TB DNA; Set 2 shows the electrophoretogram of the chip after 13-plex and 74-plex amplifications and library construction when the composition (including concentration by weight) of the additive combination was 0.3% of TMAC, 0.01% of BKB, and 8% of BSA and the nucleic acid template was 0.1 ng of TB DNA; Set 3 shows the electrophoretogram of the chip after 13-plex and 74-plex amplifications and library construction when the composition of the additive combination was 0.3% of TMAC, 0.01% of BKB, and 0.5% of DMSO and the nucleic acid template was 0.1 ng of TB DNA; Set 4 shows the electrophoretogram of the chip after 13-plex and 74-plex amplifications and library construction when no additives were added and the nucleic acid template was 0.1 ng of TB DNA. It can be seen from the above results that the additive combination containing 0.3% of TMAC, 0.01% of BKB, and 0.2% of Theist exhibited the best amplification effects. The results of 74-plex amplification and library construction are shown in Table 10 and FIG. 2:

**Table 10**

| Region for amplification by primers | 1 | 2 | 3 | 4 | Region for amplificati on by primers | 1 | 2 | 3 | 4 |
|---|---|---|---|---|---|---|---|---|---|
| rrs-2 | 4.967 9 | 4.965 4 | 4.965 4 | 4.966 6 | rpsL-1 | 3.303 4 | 3.404 7 | 3.843 9 | 3.907 3 |
| ethA-8 | 4.711 5 | 4.728 5 | 4.994 6 | 4.994 6 | ethA-4 | 3.293 8 | 3.491 1 | 4.245 6 | 4.339 7 |
| rrs-1 | 4.685 1 | 4.777 5 | 4.948 8 | 4.948 7 | rpoB-2 | 3.278 5 | 3.344 2 | 3.512 4 | 3.643 5 |
| embB-5 | 4.578 9 | 4.707 5 | 4.992 2 | 4.993 3 | ddn | 3.200 9 | 3.241 3 | 3.841 8 | 4.024 0 |
| ethA-5 | 4.545 5 | 4.723 1 | 4.995 4 | 4.995 4 | eis-2 | 3.081 7 | 3.290 7 | 4.124 3 | 4.117 9 |
| pncA-3 | 4.475 8 | 4.651 2 | 4.989 2 | 4.987 0 | katG-9 | 3.073 0 | 3.250 7 | 3.886 1 | 3.904 9 |
| katG-2 | 4.429 1 | 4.583 6 | 4.782 0 | 4.843 8 | thyA-3 | 3.067 4 | 3.269 5 | 4.091 4 | 4.157 0 |
| fabG1-1 | 4.428 7 | 4.523 8 | 4.995 7 | 4.995 7 | alr-2 | 3.054 6 | 3.147 4 | 4.010 6 | 4.115 3 |
| rpoB-3 | 4.374 0 | 4.459 3 | 4.994 0 | 4.993 6 | folC-1 | 3.037 0 | 3.141 1 | 4.159 2 | 4.203 8 |
| rpoB-3 | 4.374 0 | 4.459 3 | 4.994 0 | 4.993 6 | katG-1 | 3.003 5 | 3.283 5 | 4.574 8 | 4.443 2 |
| tlyA-4 | 4.300 9 | 4.446 2 | 4.702 1 | 4.824 6 | gyrA | 2.966 6 | 3.079 5 | 3.700 7 | 3.701 6 |
| thyA-1 | 4.215 2 | 4.375 2 | 4.947 5 | 4.929 3 | gid-3 | 2.957 6 | 3.173 5 | 3.756 6 | 3.809 8 |
| ethA-6 | 4.094 2 | 4.202 6 | 4.800 0 | 4.889 9 | fbiA | 2.718 5 | 2.673 0 | 3.329 2 | 3.478 4 |
| eis | 4.068 0 | 4.189 9 | 4.859 9 | 4.859 3 | katG-10 | 2.703 3 | 2.900 4 | 3.522 1 | 3.667 4 |
| folC-3 | 3.964 8 | 4.053 0 | 4.661 3 | 4.727 0 | ald | 2.683 9 | 2.812 2 | 2.810 9 | 2.885 4 |
| ethA-7 | 3.959 1 | 4.122 8 | 4.787 a | 4.847 9 | katG-5 | 2.658 0 | 2.848 2 | - 3.939 3 | 3.804 1 |
| gyrB-1 | 3.938 4 | 4.032 7 | 4.554 5 | 4.598 1 | pncA-4 | 2.642 5 | 2.792 4 | 3.595 3 | 3.688 2 |
| embB-1 | 3.912 3 | 4.028 7 | 4.704 8 | 4.769 2 | ethA-3 | 2.624 3 | 2.714 3 | 2.869 2 | 2.952 8 |
| thyA-2 | 3.906 6 | 4.066 0 | 4.990 3 | 4.991 3 | fabG1-2 | 2.596 6 | 2.809 6 | 3.897 6 | 3.859 0 |
| rrl-1 | 3.901 0 | 4.082 0 | 4.995 6 | 4.995 5 | ethA-1 | 2.595 5 | 2.856 1 | 4.070 3 | 4.051 4 |
| gyrB-2 | 3.888 5 | 3.998 0 | 4.534 5 | 4.567 7 | rpoB-1 | 2.571 7 | 2.779 6 | 3.846 6 | 3.789 9 |
| ribd | 3.884 9 | 4.016 8 | 4.899 5 | 4.993 3 | katG-3 | 2.477 1 | 2.627 4 | 3.957 7 | 3.889 5 |
| katG-8 | 3.867 3 | 3.990 4 | 4.694 9 | 4.735 6 | embB-4 | 2.444 0 | 2.431 4 | 3.117 6 | 3.206 0 |
| embA | 3.778 9 | 3.950 5 | 4.081 3 | 4.128 0 | embB-2 | 2.406 5 | 2.673 0 | 3.389 2 | 3.409 3 |
| katG-7 | 3.695 4 | 3.826 7 | 4.525 3 | 4.653 6 | ethA-2 | 2.390 9 | 2.482 9 | 2.328 4 | 2.488 6 |
| katG-4 | 3.691 4 | 3.895 4 | 4.961 1 | 4.959 6 | folC-2 | 2.342 4 | 2.521 1 | 3.772 9 | 3.766 3 |
| rrs-3 | 3.689 5 | 4.121 1 | 4.746 7 | 4.862 9 | gid-2 | 2.255 3 | 2.433 0 | 3.167 0 | 3.245 3 |
| embB-3 | 3.674 0 | 3.777 1 | 4.062 4 | 4.207 2 | pncA-2 | 2.220 1 | 2.195 9 | 2.947 4 | 3.004 8 |
| inhA | 3.634 8 | 3.807 5 | 4.703 0 | 4.757 6 | tlyA-1 | 2.041 4 | 2.049 2 | 3.001 3 | 2.925 3 |
| thyA-5 | 3.619 0 | 3.949 6 | 4.995 2 | 4.994 6 | rplC | 2.004 3 | 2.334 5 | 3.444 4 | 3.371 6 |
| thyA-4 | 3.608 8 | 3.845 3 | 4.956 2 | 4.989 6 | tlyA-5 | 1.963 8 | 2.292 3 | 3.248 0 | 3.292 0 |
| tlyA-3 | 3.584 2 | 3.727 8 | 4.550 7 | 4.594 2 | gid-1 | 1.913 8 | 1.991 2 | 2.984 1 | 2.982 7 |
| alr-1 | 3.578 3 | 3.693 6 | 4.257 2 | 4.388 3 | katG-6 | 1.491 4 | 1.623 2 | 2.676 7 | 2.619 1 |
| Rv0678-3 | 3.544 3 | 3.705 8 | 4.442 5 | 4.468 7 | pncA-1 | 1.301 0 | 1.662 8 | 2.667 5 | 2.656 1 |
| rpsL | 3.518 8 | 3.643 6 | 4.760 9 | 4.900 2 | alr-3 | 1.176 1 | 1.113 9 | 2.033 4 | 2.257 7 |
| gid-4 | 3.435 7 | 3.579 0 | 4.198 1 | 4.286 1 | / | | | | |
| tlyA-2 | 3.334 7 | 3.498 3 | 4.331 0 | 4.354 1 | | | | | |
| Rv0678-4 | 3.321 6 | 3.453 6 | 3.739 8 | 3.822 8 | | | | | |
| Rv0678-2 | 3.303 8 | 3.472 9 | 4.280 9 | 4.293 9 | | | | | |

Table 10 shows the number of reads by sequencing after the amplifications with 74 specific primer pairs and no additives or three additives on corresponding amplification regions in different samples. Due to the great number of reads, the logarithms of the numbers were compared. The sequencing depths were summarized and compared (the sequencing depth refers to the number of aligned reads with the corresponding target sequences after aligning all reads obtained by sequencing with target regions in a reference sequence (74 target sequence fragments in H37Rv)).

In order to more intuitively illustrate the sequencing depth results after PCR amplification of the specific primers, the data in Table 10 is assigned with corresponding grayscale value according to the sizes, thus acquiring FIG. 2, where a higher grayscale value (a deeper color) indicates a more significant sequencing depth. In Table 10 and FIG. 2, reference numerals 1 and 3 denote amplifications of the same sample, where reference numeral 1 denotes a reagent with no additives, and reference numeral 3 denotes a reagent with three additives (0.3% of TMAC, 0.01% of BKB, and 0.2% of Theist); reference numerals 2 and 4 denote amplifications of another sample, where reference numeral 2 denotes a reagent with no additives, and reference numeral 4 denotes a reagent with three additives (0.3% of TMAC, 0.01% of BKB, and 0.2% of Theist). As shown in FIG. 2, the depths of the groups with additives (examples) were significantly higher than those of the groups with no additives (comparative examples) in various samples, suggesting that the additive combination selected in the examples of the present disclosure has a significant promoting effect on multiplex PCR amplification.

In conclusion, the additive for PCR amplification provided by the present application has a promoting effect on PCR amplification, and is beneficial to improving the amplification efficiency and amplification specificity of PCR reactions and prospective in multiplex PCR amplification.

The applicant declares that the above description is only intended to illustrate the specific embodiments of the present disclosure, rather than limit the scope of the present disclosure. It will be appreciated by those skilled in the art that any changes or substitutions that can be easily obtained by those skilled in the art within the technical scope of the present disclosure disclosed herein fall within the protection scope and disclosure scope of the present disclosure.

## Claims

1. An additive for PCR amplification, comprising benzalkonium bromide.

2. The additive for PCR amplification according to claim 1, wherein benzalkonium bromide is present at a concentration of 0.001-0.1% by weight in a PCR amplification system.

3. The additive for PCR amplification according to claim 1 or 2, further comprising tetramethylammonium chloride and/or tetrapolyethylene glycol monolauryl ether.

4. The additive for PCR amplification according to claim 3, further comprising at least one of bovine serum albumin, polyethylene glycol, or dimethyl sulfoxide.

5. The additive for PCR amplification according to claim 4, wherein the additive is a combination of benzalkonium bromide and tetramethylammonium chloride.

6. The additive for PCR amplification according to any one of claims 1-4, wherein the additive is present at concentrations of 0.001-0.1% for benzalkonium bromide and 0.1-0.5% for tetramethylammonium chloride by weight in the PCR amplification system.

7. The additive for PCR amplification according to any one of claims 1-4, wherein the additive is a combination of benzalkonium bromide and tetrapolyethylene glycol monolauryl ether.

8. The additive for PCR amplification according to claim 7, wherein the additive is present at concentrations of 0.001-0.1% for benzalkonium bromide and 0.001-0.2% for tetrapolyethylene glycol monolauryl ether by weight in the PCR amplification system.

9. The additive for PCR amplification according to any one of claims 1-4, wherein the additive is a combination of benzalkonium bromide, tetramethylammonium chloride, and tetrapolyethylene glycol monolauryl ether.

10. The additive for PCR amplification according to claim 9, wherein the additive is present at concentrations of 0.001-0.1% for benzalkonium bromide, 0.1-0.5% for tetramethylammonium chloride, and 0.001-0.2% for tetrapolyethylene glycol monolauryl ether by weight in the PCR amplification system.

11. The additive for PCR amplification according to any one of claims 1-10, wherein the additive is for use in multiplex PCR amplification.

12. A multiplex PCR amplification kit, comprising the additive for PCR amplification according to any one of claims 1-11.

13. The multiplex PCR amplification kit according to claim 12, further comprising a primer composition.

14. The multiplex PCR amplification kit according to claim 13, wherein the primer composition comprises one or more primer pairs, and the primers are selected from the nucleotide sequences set forth in SEQ ID NOs. 1-174.

15. A method for detecting drug resistance in M. *tuberculosis,* comprising:
performing multiplex PCR amplification on a M. *tuberculosis* culture using the multiplex PCR amplification kit according to any one of claims 12-14;
sequencing the amplification product; and
determining the drug resistance of M. *tuberculosis* based on the sequencing result.
